# EUROPEAN PATENT SPECIFICATION

(11) **EP 1 092 441 B1**
(45) Date of publication and mention of the grant of the patent: **01.02.2006**
(21) Application number: 99957624.2
(22) Date of filing: 29.06.1999
(51) Int. Cl.: A61M 5/315

(54) **CHARGED SYRINGE GASKET AND CHARGED SYRINGE**
DICHTUNG FÜR EINE GEFÜLLTE SPRITZE
JOINT DE SERINGUE CHARGEE ET SERINGUE CHARGEE

(30) Priority: 29.06.1998 JP 18300598
(43) Date of publication of application: 18.04.2001
(73) Proprietor: BRACCO International B.V., 1077 ZX Amsterdam (NL); DAIKYO SEIKO, LTD., Tokyo (JP)
(72) Inventor: YANASE, Kazuyuki, Fukaya-shi, Saitama 366-0035 (JP); NAKAMOTO, Keizou, Eisai Co., Ltd., Honjyo-shi, Saitama 367-0048 (JP); IIJIMA, Kazumi, Isesaki-shi, Gumma 372-0812 (JP)
(74) Representative: Turi, Michael
(86) International application number: PCT/JP1999/003459
(87) International publication number: WO 2000/000243

(56) References cited:
- EP-A- 0 372 549
- EP-A- 0 497 567
- EP-A- 0 768 093
- WO-A-98/17338
- JP-A- 7 184 999
- JP-B2- 4 003 777
- JP-B2- 7 047 045
- US-A- 4 997 423
- US-A- 5 411 489

## Description

### Field of the Invention

The present invention relates to a gasket for a pre-filled syringe and the pre-filled syringe.

### Background Art

In generally, chemical liquid is charged into a syringe barrel when it is used. In recent years, a pre-filled syringe in which chemical liquid is previously charged into the syringe barrel has been developed, and working load in medical site is reduced. Recently, a pre-filled syringe in which contrast medium is charged into the syringe is also used. Since the contrast medium has relatively high viscosity, it is considered that it is important to increase sliding ability of a gasket so as to reduce the charging pressure. Especially when a syringe barrel is made of polymeric resin, since the sliding ability of the gasket is inferior, silicon oil is applied onto a peripheral side surface of a gasket that is in contact with an inner surface of the syringe barrel to enhance the sliding ability, or polyethylene fluoride resin such as Teflon (registered trade name) is laminated. However, if silicon applied to a gasket is mixed into chemical liquid, it becomes foreign matter, this may cause product defect, and there is apprehension that such foreign matter may have toxicity to human body.

Prior art document US-A-4 997 423 recognises the problems in connection with silicone oils used as a lubricant for syringes. Furthermore, this document (see figures 1 to 5) teaches to improve the sliding properties of the rubber-like elastic stopper base by fully laminating the distal surface of said base which is in contact with the liquid medicament and fully laminating the circumferential surface of said base which slides on the inner wall of the barrel.

It is normal that the pre-filled syringe is sterilized after chemical liquid is charged or chemical liquid is charged in axenic conditions. Sterilization after charging is carried by heating the pre-filled syringe for example, but it is important to form the gasket into such a shape that high pressure chemical liquid caused by heat at the time of sterilization is not leaked. When polyethylene fluoride resin or the like is laminated on a gasket, since the polyethylene fluoride resin is hard, if the gasket is inserted into the syringe barrel, fine wrinkles may be generated on a peripheral side surface of the gasket, and chemical liquid may be leaked through the wrinkles at the time of sterilization.

The present inventor studies hard to solve these problems and as a result, the inventor invented the following inventions.

### Summary of the Invention

According to claim 1, there is provided a gasket used for a pre-filled syringe into which liquid is charged, wherein a peripheral side surface of the gasket that is in contact with an inner surface of the syringe barrel is provided with a restriction, and a periphery of a bottom surface of the gasket that is not in contact with the liquid is formed into a tapered shape whereby only the said peripheral side surface that is in contact with an inner surface of the syringe barrel is laminated with polyethylene fluoride resin.

In this gasket of claim 1, as described in claim 2, it is preferable that hardness of the gasket is 55 to 60 when the hardness is measured by a JIS hardness meter. Further, as described in claim 3, it is preferable that the peripheral side surface that is in contact with the inner surface of the syringe barrel and/or a bottom surface that is in contact with liquid is laminated with polyethylene fluoride resin.

According to claim 4, there is provided a pre-filled syringe into which liquid is charged and tightly closed with the gasket described in any one of claims 1 to 3.

In this pre-filled syringe of claim 4, the liquid is, for example, contrast medium as described in claim 5.

In the gasket of claims 1 to 3 and the pre-filled syringe of claims 4 and 5, the gasket plays a role as a lid for tightly closing the liquid charged into the syringe barrel, and also plays a role as a piston when the pre-filled syringe is used. As the JIS hardness meter for measuring the hardness of the gasket, "Durometer" produced by Shimazu Seisakusho can be used for example. A preferable range of the hardness of the gasket measured by the JIS hardness meter is 57 to 59. Material of the gasket is not limited only if it has appropriate hardness, but usually, the material is normal butyl rubber, silicon rubber or polymeric resin, and more preferably, chlorinated butyle rubber.

The peripheral side surface that is in contact with the syringe barrel or the bottom surface that is in contact with liquid can be laminated with polyethylene fluoride resin using a conventionally known laminating method. Silicon may be applied to the peripheral side surface of the gasket that is in contact with the inner surface of the syringe barrel, but it is preferable that silicon is not applied to the bottom syringe that is in contact with the liquid charged into the syringe barrel. The silicon can also be applied by a conventionally known application method.

Material of the syringe barrel is not limited, and any of glass and resin can be used. Especially, resin is preferable, and example of the material is annular polyolefin fiber.

Liquid to be charged into the syringe barrel is not limited to the contrast medium, and liquid other than the contrast medium may be used. Example of contrast medium is iomeprole. An amount of liquid to be charged into the syringe barrel is usually about 10 to 200 ml, and more preferably 50 to 100 ml.

### Brief Description of the Drawings

Fig. 1 is a sectional view of a pre-filled syringe according to an embodiment of the present invention; and
Fig. 2 is a side view of a gasket according to the embodiment of the invention.

### Detailed Description of the Preferred Embodiments

A preferred embodiment of the present invention will be explained with reference to the accompanying drawings below.

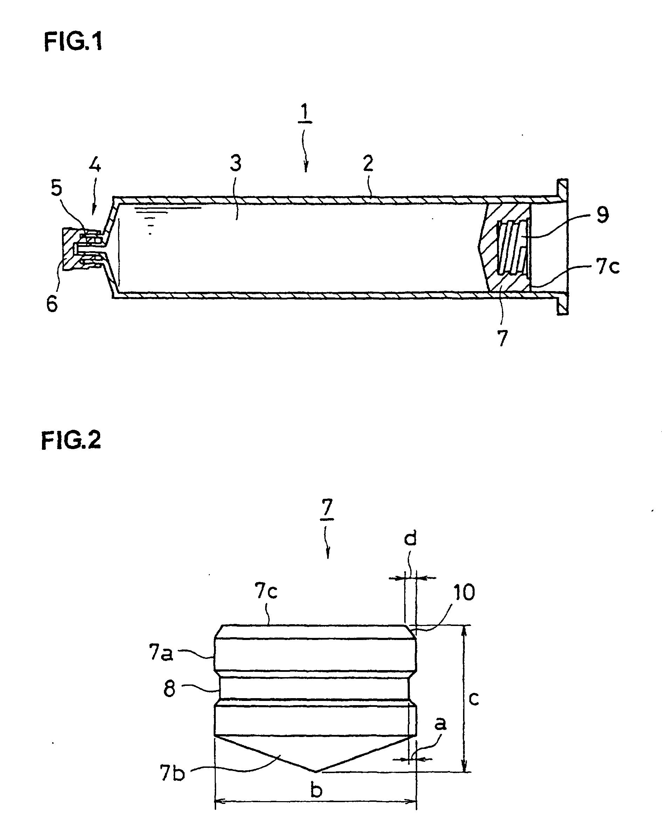

As shown in Fig.1, in a pre-filled syringe 1 of this embodiment, a contrast medium 3 as one example of chemical liquid is charged in a cylindrical syringe barrel 2. The syringe barrel 2 is made of annular polyolefin fiber for example. The syringe barrel 2 is provided at its tip end (left end of the syringe barrel 2 in Fig.1) with a nozzle 4. The nozzle 4 is formed with a lure lock 5, and by mounting a cap 6 on the lure lock 5, the nozzle 4 is tightly closed. A columnar gasket 7 is inserted into the syringe barrel 2 from an opened rear end (right end of the syringe barrel 2 in Fig.1) of the syringe barrel 2, and the contrast medium 3 in the syringe barrel 2 is tightly closed by the gasket 7.

The gasket 7 is made of chlorinated butyle rubber for example, and the gasket 7 preferably has hardness of 55 to 60 degrees, more preferably 57 to 59 degrees when the hardness is measured using JIS hardness meter. As shown in Fig.2, a peripheral side surface 7a of the gasket 7 that is in contact with an inner surface of the syringe barrel 2 is formed with an annular restriction 8. A depth a of the restriction 8 is about 0.05 to 1.0 mm, and more preferably, 0.1 to 0.5 mm if the gasket has a diameter b of 30 to 35 mm and a height c of 15 to 18 mm.

A bottom surface 7b (lower surface of the gasket 7 in Fig.2) that is in contact with the contrast medium 3 charged into the syringe barrel 2 is formed into a conical surface. The bottom surface 7b and the peripheral side surface 7a are laminated with polyethylene fluoride resin.

A central portion of a bottom surface 7c (upper surface of the gasket 7 in Fig.2) that is not in contact with the contrast medium 3 charged into the syringe barrel 2 is formed, as shown in Fig.2, with a threaded hole 9 into which a rod is fitted. As shown in Fig.2, a periphery of the bottom surface 7c is formed into a tapered slant 10. A range (range of the gasket 7 from an outer diameter in its diametrical direction) d where the slant 10 is formed is about 0.5 to 5 mm, and more preferably about 1 to 3 mm if the gasket has a diameter b of 30 to 35 mm and a height c of 15 to 18 mm.

According to this pre-filled syringe 1 having the above-described structure, the cap 6 is removed from the nozzle 4, and a tip end of an extension tube (not shown) for example is threadedly fitted to the lure lock 5. A rod (not shown) is fitted to the bottom surface 7c of the gasket 7. Then, the rod is pushed to push out the contrast medium 3 in the syringe barrel 2 through the extension tube, thereby charging the contrast medium 3 into a target position.

### Industrial Applicability

According to the inventions of claims 1 to 5, the sliding ability when polyolefin resin is used as material of a syringe barrel is used is remarkably enhanced, and liquid is not leaked almost at all when the pre-filled syringe is sterilized. The gasket can smoothly move at the time of sterilization even when the gasket is pushed out, and there is no adverse possibility that the gasket is diagonally inclined with respect to a center axis of the syringe barrel.

### DESCRIPTION OF CHARACTERS

- 1: pre-filled syringe
- 2: syringe barrel
- 3: contrast medium
- 4: nozzle
- 5: lure lock
- 6: cap
- 7: gasket
- 7a: peripheral side surface
- 7b ,7c: bottom surface
- 8: annular restriction
- 9: threaded hole
- 10: tapered slant

## Claims

1. A gasket (7) used for a pre-filled syringe (1) into which liquid is charged, wherein a peripheral side surface (7a) of the gasket (7) that is in contact with an inner surface of the syringe barrel (2) is provided with a restriction (8) and a periphery of a bottom surface (7c) of the gasket (7) that is not in contact with the liquid is formed into a tapered shape (10), **characterized in that** only the peripheral side surface (7a) that is in contact with an inner surface of the syringe barrel (2) is laminated with polyethylene fluoride resin.

2. A gasket according to claim 1, wherein hardness of the gasket (7) is 55 to 60 when the hardness is measured by a JIS hardness meter.

3. A gasket according to claim 1, wherein a bottom surface (7b) that is in contact with liquid is laminated with polyethylene fluoride resin.

4. A pre-filled syringe into which liquid is charged and tightly closed with the gasket (7) described in any one of claims 1 to 3.

5. A pre-filled syringe according to claim 4, wherein the liquid is contrast medium.

## Patentansprüche

1. Eine Dichtung (7), die verwendet wird für eine zuvor gefüllte Spritze (1), in die Flüssigkeit gefüllt wurde, wobei eine umlaufende Seitenoberfläche (7a) der Dichtung (7), die in Kontakt mit einer inneren Oberfläche des Spritzenrohres (2) ist, mit einer Einschnürung (8) versehen ist und ein Umfang einer Bodenoberfläche (7c) der Dichtung (7), der nicht in Kontakt mit der Flüssigkeit ist, zu konischer Form (10) ausgebildet ist, **dadurch gekennzeichnet, daß** nur die umlaufende Seitenoberfläche (7a), die in Kontakt mit einer inneren Oberfläche des Spritzenrohres (2) ist, mit Polyethylenfluoridharz beschichtet ist.

2. Dichtung nach Anspruch 1, bei welcher die Härte der Dichtung (7) 55-60 ist, wenn die Härte von einem JIS-Härtemesser gemessen wird.

3. Dichtung nach Anspruch 1, bei welcher eine Bodenoberfläche (7b), die in Kontakt mit einer Flüssigkeit ist, mit Polyethylenfluoridharz beschichtet ist.

4. Zuvor gefüllte Spritze, in die Flüssigkeit gefüllt ist und die mit der in einem der Ansprüche 1-3 beschriebenen Dichtung (7) dicht verschlossen ist.

5. Zuvor gefüllte Spritze nach Anspruch 4, bei welcher die Flüssigkeit Kontrastmittel ist.

## Revendications

1. Joint (7) utilisé pour une seringue préremplie (1) dans laquelle on charge du liquide, dans lequel une surface latérale périphérique (7a) du joint (7) qui est en contact avec une surface interne du corps (2) de la seringue, est prévue avec une restriction (8) et une périphérie d'une surface inférieure (7c) du joint (7) qui n'est pas en contact avec le liquide, est formée selon une forme progressivement rétrécie (10), **caractérisé en ce que** seule la surface latérale périphérique (7a) qui est en contact avec une surface interne du corps (2) de la seringue est laminée avec de la résine à base de fluorure de polyéthylène.

2. Joint selon la revendication 1, dans lequel la dureté du joint (7) est de 55 à 60 lorsque la dureté est mesurée par un duromètre JIS.

3. Joint selon la revendication 1, dans lequel une surface inférieure (7b) qui est en contact avec du liquide, est laminée avec de la résine à base de fluorure de polyéthylène.

4. Seringue préremplie dans laquelle on charge du liquide et qui est hermétiquement fermée avec le joint (7) décrit dans l'une quelconque des revendications 1 à 3.

5. Seringue préremplie selon la revendication 4, dans laquelle le liquide est un produit de contraste.
